Europäisches Patentamt

European Patent Office

Office européen des brevets

(11) Veröffentlichungsnummer: **0 120 235**
**A1**

## (12) EUROPÄISCHE PATENTANMELDUNG

(21) Anmeldenummer: **84101166.1**

(22) Anmeldetag: **06.02.84**

(51) Int. Cl.³: **A 61 F 13/20**
**A 61 F 11/00**

(30) Priorität: **01.03.83 CH 1123/83**

(43) Veröffentlichungstag der Anmeldung:
**03.10.84 Patentblatt 84/40**

(84) Benannte Vertragsstaaten:
**AT BE DE FR GB IT LU NL SE**

(71) Anmelder: **Fassbind & Fassbind**
**Appisbergstrasse 20**
**CH-8708 Männedorf(CH)**

(72) Erfinder: **Fassbind, Karl**
**Appisbergstrasse 20**
**CH-8708 Männedorf(CH)**

(74) Vertreter: **Blum, Rudolf Emil Ernst et al,**
**c/o E. Blum & Co Patentanwälte Vorderberg 11**
**CH-8044 Zürich(CH)**

(54) Wattestäbchen.

(57) Das Wattestäbchen hat am Ende die bei den üblichen, bekannten Wattestäbchen vorhandene Form (3) des Wattepolsters (2), sowohl im Durchmesser als auch in der halbkugelförmigen Stirnfläche. Daran schliesst sich eine im Durchmesser verdickte Sicherheitspartie (4) des Wattepolsters (2) an. Diese Sicherheitspartie (4) ist dem mittleren Bereich (1) des Wattestäbchens zugewandt. Das gesamte Wattepolster (2) hat hierdurch die Form einer balligen Flasche (3, 4, 5). Die Flaschenkopfpartie (3) dient zum Reinigen z.B. von Ohren und Nase, und die im Durchmesser dickere Flaschenbauchpartie (4) bildet die Sicherheitspartie, die ein zu tiefes Eindringen des Wattestäbchens beim Reinigen verhindert. Hierdurch können Verletzungen vermieden werden.

- 1 -

Wattestäbchen
------------

Die Erfindung betrifft ein Wattestäbchen, bei dem ein Tragschaft an zumindest einem Ende mit einem Wattepolster versehen ist.

Solche Wattestäbchen haben sich auf dem Markt für verschiedene Reinigungszwecke sehr stark verbreitet durchgesetzt. Sie werden zum Beispiel für die Hygiene von Ohren und Nase verwendet, wobei wahrscheinlich der verbreiteste Verwendungszweck beim Reinigen des Gehörgangs liegt. Dies ist mit den bekannten Wattestäbchen problematisch, da ein zu tiefes Eindringen des Wattepolsters in den Gehörgang erheblichen Schaden verursachen kann. Es ist hierbei nicht nur an ein Verletzen des Trommelfelds gedacht, sondern durch ein zu tiefes Eindringen des Wattepolsters in den Gehörgang kann auch Ohrenschmalz tief ins Innere des Gehörgangs geschoben werden und verursacht hierdurch Störungen, wobei dann ein solcher nach innen geschobener Ohrenschmalzpfropfen vom Arzt durch Wasserspülungen mit verhältnismässig hohem Druck herausgespült werden muss. Es sind aber auch schon häufig Verletzungen des inneren Gehörgangs und des Trommelfells durch zu tiefes Eindringen des Wattepolsters in den Gehörgang entstanden.

Es wird die Schaffung eines Wattestäbchens bezweckt, mit dem die vorerwähnten Nachteile vermieden werden können.

Das erfindungsgemässe Wattestäbchen ist dadurch gekennzeichnet, dass das Wattepolster flaschenförmig ist, wobei die im Durchmesser kleinere Flaschenkopfpartie beim Tragschaftende liegt und die im Durchmesser grössere Flaschenbauchpartie dem mittleren Bereich des Tragschaftes zugewandt ist.

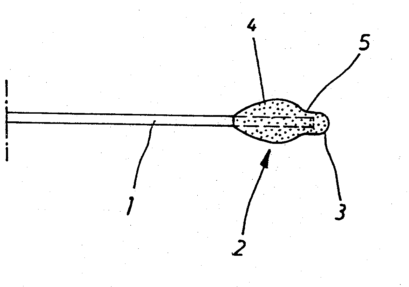

In der Zeichnung ist ein Ausführungsbeispiel des Erfindungsgegenstandes in Seitenansicht dargestellt.

Das Wattestäbchen hat einen Tragschaft 1, der an zumindest einem Ende mit einem Wattepolster 2 versehen ist. Letzteres hat die Form einer bauchigen Flasche oder eines beim Kegelsport verwendbaren Kegels. Das Wattepolster 2 hat eine im Durchmesser kleinere Flaschenkopfpartie 3 und eine im Durchmesser grössere Flaschenbauchpartie 4. Die Flaschenkopfpartie 3 liegt am Ende des Wattestäbchens, sozusagen an seiner einen Spitze und hat in üblicher Weise eine halbkugelförmige Stirnfläche. Die Flaschenbauchpartie 4 ist dem mittleren Bereich des Tragschaftes 1 zugewandt. Durch die aus der Zeichnung ersichtliche Form des Wattepolsters kann man sagen, dass die Flaschenkopfpartie 3 und die Flaschenbauchpartie 4 ballig sind. Zwischen diesen beiden Partien 3 und 4 liegt eine gekrümmte Flaschenhalspartie 5.

Die Flaschenkopfpartie 3 hat im Durchmesser und mit seiner halbkugelförmigen Stirnfläche die Form eines üblichen, bekannten Wattestäbchens in seinem Spitzenbereich. Der Durchmesser dieser Flaschenkopfpartie 3 ist also derart, dass man mit ihr in der üblichen Weise zum Beispiel in den Gehörgang eindringen kann. Die Flaschenbauchpartie 4 dagegen dient als Sicherheitspartie, ist also im Durchmesser so gross, dass diese Flaschenbauchpartie 4 nicht ganz in den Gehörgang eintreten kann. Durch die ballige Form dieser Flaschenbauchpartie kann also das Wattestäbchen nur mit seiner Partie 3, 5 und einem daran anschliessenden anfänglichen Bereich der Flaschenbauchpartie 4 in den Gehörgang eindringen. Der weitere, im Durchmesser grösser werdende Bereich der Flaschenbauchpartie 4 verhindert ein tieferes Eindringen.

Durch die als Sicherheitspartie dienende Flaschenbauchpartie 4 wird somit ein zu tiefes Eindringen des Wattestäbchens zum Beispiel in den Gehörgang und in die Nase wirksam und schmerzlos verhindert, so dass hierdurch Verletzungen und das eingangs genannte tiefe Einstossen eines Ohrenschmalzpfropfens vermieden werden können. Natürlich können hierdurch auch Verletzungen im Innern der Nase verhindert werden, falls das Wattestäbchen für die Hygiene der Nase verwendet wird. Durch die Flaschenbauchpartie 4 entsteht somit ein eigentliches Sicherheits-Wattestäbchen, mit dem sich auch Kinder nicht mehr so leicht Verletzungen beibringen können.

Patentansprüche
----------------

1. Wattestäbchen, bei dem ein Tragschaft (1) an zumindest einem Ende mit einem Wattepolster (2) versehen ist, dadurch gekennzeichnet, dass das Wattepolster (2) flaschenförmig ist, wobei die im Durchmesser kleinere Flaschenkopfpartie (3) beim Tragschaftende liegt und die im Durchmesser grössere Flaschenbauchpartie (4) dem mittleren Bereich des Tragschaftes (1) zugewandt ist.

2. Wattestäbchen nach Anspruch 1, dadurch gekennzeichnet, dass die Flaschenkopfpartie (3) und die Flaschenbauchpartie (4) ballig sind und zwischen diesen beiden Partien eine gekrümmte Flaschenhalspartie (5) liegt.

3. Wattestäbchen nach Anspruch 1, dadurch gekennzeichnet, dass das flaschenförmige Wattepolster (2) die Form eines beim Kegelsport verwendbaren Kegels (3,4, 5) hat.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| Y | US-A-2 876 501 (GLICKSTON)<br>* Figur 1 * | 1 | A 61 F 13/20<br>A 61 F 11/00 |
| A | | 2,3 | |
| | --- | | |
| Y | FR-A-2 277 557 (CHOPPY)<br>* Seite 1, Zeilen 19-26 * | 1 | |
| | --- | | |
| A | US-A-2 510 961 (DAVIS) | | |
| | --- | | |
| A | US-A-2 006 539 (DEFORD) | | |
| | ----- | | |

**RECHERCHIERTE SACHGEBIETE (Int. Cl. ³)**

A 45 D 44/00
A 47 K 7/00
A 61 F 11/00
A 61 F 13/00

Der vorliegende Recherchenbericht wurde für alle Patentansprüche erstellt.

| Recherchenort | Abschlußdatum der Recherche | Prüfer |
|---|---|---|
| BERLIN | 24-05-1984 | KANAL P K |